# EUROPÄISCHE PATENTSCHRIFT

(11) **EP 0 855 434 B1**
(45) Veröffentlichungstag und Bekanntmachung des Hinweises auf die Patenterteilung: **14.06.2006**
(21) Anmeldenummer: 98250017.5
(22) Anmeldetag: 20.01.1998
(51) Int. Cl.: C09J 4/02, C09J 11/06, A61K 6/00

(54) **Haftvermittelnde Zusammensetzung für Verbunde zwischen Kunststoff und anderen Werkstoffen**
Adhesive compositon for adhesion of plastics to other materials
Composition adhésive pour fixer des matériaux plastiques à d'autres matériaux

(30) Priorität: 25.01.1997 DE 19702704
(43) Veröffentlichungstag der Anmeldung: 29.07.1998
(73) Patentinhaber: Ivoclar Vivadent AG, 9494 Schaan (LI)
(72) Erfinder: Göbel, Roland, 07749 Jena (DE)
(74) Vertreter: UEXKÜLL & STOLBERG

(56) Entgegenhaltungen:
- EP-A- 0 017 937
- EP-A- 0 103 420
- EP-A- 0 507 177
- GB-A- 772 675

## Beschreibung

Die Erfindung betrifft eine haftvermittelnde Zusammensetzung für Verbunde zwischen Kunststoff und anderen Werkstoffen, die sich zum dauerhaften und feuchtigkeitsstabilen Verbinden von insbesondere Dentalkunststoffen mit Dentallegierungen eignet.

Zur optimalen ästhetischen Gestaltung prothetischer Metallgerüste, wie Kronen und Brücken, ist es notwendig, die Metalloberfläche mit einem zahnfarbenen Kunststoff zu versehen. Der dabei eingesetzte Kunststoff setzt sich üblicherweise aus einer organischen Matrix auf Basis von Methacrylat- und Dimethacrylatmonomeren, einem Initiatorsystem, einem organischen Füllstoff sowie Farbpigmenten zusammen. Wegen der extremen Bedingungen des Mundmilieus, der stetigen Feuchtigkeits- und Temperaturwechsel sowie der hohen mechanischen Belastung sind mechanische Verankerungen zur Befestigung des Kunststoffes nicht ausreichend, um auf Dauer einen auch nur annähernd zufriedenstellenden Verbund zwischen der Dentallegierung und dem Verblendkunststoff aufrecht zu erhalten. Es kommt daher schon nach kurzer Zeit in der Regel zu einer Randspaltbildung von 5 bis 10 µm zwischen der Legierung und dem Kunststoff, wodurch eine deutliche Schwächung des Verbundes bis hin zum Ablösen der Verblendung auftreten kann. Die Ablösung ermöglicht im Spaltbereich eine Korrosion des Metalles und mit dieser tritt ein unästhetisches Verfärben des Verblendkunststoffes auf.

Zum spaltfreien Verbinden von Dentalkunststoffen mit Dentallegierungen wurden in den letzten Jahren zahlreiche Vorschläge gemacht. Grundprinzip eines Teils dieser Verfahren ist es, daß in einem ersten Schritt eine silikatische Schicht auf die Metalloberfläche aufgebracht (Silikatisierung) und in einem zweiten Schritt die Oberfläche mit einem funktionellen Alkoxysilan bestrichen wird (Silanisierung). Dabei wirkt das Alkoxysilan, meist hydrolysiertes Methyacryloyloxypropyltrimethoxysilan, als Bindeglied zwischen der anorganischen silikatischen Schicht und dem in der Regel methacrylathaltigen Dentalkunststoff. Diese Wirkung beruht darauf, daß einerseits die OH-Gruppen des Silans mit den OH-Gruppen an der Oberfläche der Silikatschicht durch eine Kondensationsreaktion an die Silikatschicht chemisch gebunden werden und andererseits über die Methacrylatgruppe des Silans eine Anbindung an die Matrix des Kunststoffes erfolgt. Die bekannten Verfahren unterscheiden sich dabei durch die Art des Aufbringens der Silikatschicht, während das Aufbringen des Haftsilans bei allen Verfahren nahezu identisch ist.

In der US-A-4,364,731 wird ein Verfahren zum Aufbringen einer Siliciumdioxidschicht auf metallische Dentalprothesenteile durch eine Hochfrequenz-Magnetron-Sputtervorrichtung beschrieben.

In der DE-C-34 03 894 wird ein Verfahren offenbart, bei dem die Silikatschicht durch einen Flammenhydrolyseprozeß von Tetraethoxysilan aufgebracht wird.

Weiter ist in DD-A-276 453 ein Verfahren offenbart, bei dem eine Silikat-Chromoxid-Schicht durch eine Sol-Gel-Lösung auf die Dentallegierungsoberfläche aufgebracht und durch einen anschließenden Temperprozeß bei 320°C für 2 bis 8 Minuten verfestigt wird.

In der DE-A-38 02 043 wird ein Verfahren beschrieben, bei dem die Silikatbeschichtung durch einen Korundstrahlprozeß erfolgt, indem dem Strahlkorund eine gewisse Menge Siliciumdioxid einer mittleren Teilchengröße < 5 µm zugesetzt wird. Im Aufschlagbereich der Korundteilchen treten dabei örtliche Energiedichten auf, die ausreichen, um die feinteiligen Silikatpartikel auf die metallische Oberfläche aufzuschmelzen.

Schließlich wird in der DE-A-42 28 530.5 ein Verfahren beschrieben, bei dem anstelle der anorganischen Silikatschicht eine organische Phenolharz-Acrylat-Methacrylat-Schicht als Verbundschicht dient.

In der GB 772 675 A werden Zusammensetzungen auf Basis von drei- bis fünfwertigen Metallalkoholaten offenbart, die durch Hydrolyse haftungsverbessernde Filme bilden sollen.

In der EP-A-0 017 937 werden Klebstoffzusammensetzungen für medizinische oder dentale Anwendungen beschrieben, die neben einem polymerisierbaren Acrylderivat mindestens eine Alkoxytitanverbindung oder eine Alkoxysiliciumverbindung enthalten.

Die EP-A-0 103 420 offenbart ein Beschichtungsmaterial, das ein Polymer mit einer Säurezahl von 30 bis 700 sowie eine polymerisierbare Vinylverbindung und/oder ein organisches Titanat enthält.

In der EP-A-0 507 177 werden alkoxyfunktionelle Siliconharze beschrieben, die durch Feuchtigkeit aushärten und als provisorische Befestigungsmaterialien verwendet werden.

Bei den beschriebenen Verbundverfahren, insbesondere bei dem Sputterprozeß, dem Flammenhydrolyseverfahren und dem Sol-Gel-Verfahren, ist es zur haftfesten Anbindung der Verbundschicht an die Legierungsoberfläche erforderlich, diese auf eine Temperatur von etwa 300°C zu erwärmen. Eine so hohe Temperatur erweist sich bei bestimmten Legierungen, z.B. Legierungen mit einem Kupfergehalt von > 5 % jedoch als nachteilig, da sie zur Veränderung der Legierungszusammensetzung an der Oberfläche führt. Dies hat zudem eine Verminderung der Haftung sowie eine Verfärbung der Legierung zur Folge. Weiter bedürfen die bekannten Verfahren zu ihrer Durchführung eines hohen apparativen Aufwandes oder des Einsatzes von teuren Chemikalien.

Der Erfindung liegt somit die Aufgabe zugrunde, eine haftvermittelnde Zusammensetzung für Verbunde zwischen Kunststoff und anderen Werkstoffen zur Verfügung zu stellen, die mit nur geringem apparativen Aufwand und bei geringer Temperaturbelastung des Werkstoffes eingesetzt werden kann und die Herstellung einer haftfesten und feuchtigkeitsstabilen Verbundschicht und durch diese die Erzeugung einer dauerhaften, hochfesten und randspaltfreien Verbindung zwischen Kunststoff und anderen Werkstoffen ermöglicht.

Diese Aufgabe wird durch die haftvermittelnde Zusammensetzung nach den Ansprüchen 1 bis 8 gelöst. Die Erfindung bezieht sich ebenfalls auf die Verwendung der Zusammensetzung nach den Ansprüchen 9 bis 10, ein Verfahren zur Herstellung von Verbunden zwischen Kunststoff und anderen Werkstoffen nach den Ansprüchen 11 bis 16 sowie den eine Verbundschicht aufweisenden Werkstoff nach Anspruch 17. Weiterer Gegenstand der Erfindung ist ein Verfahren zum Aufbringen einer Verbundschicht auf einen Werkstoff gemäß Anspruch 18.

Die erfindungsgemäße haftvermittelnde Zusammensetzung für Verbunde zwischen Kunststoff und anderen Werkstoffen zeichnet sich dadurch aus, daß sie (a) mindestens ein Alkoholat des Titans, des Zirkoniums oder des Hafniums und (b) mindestens ein Alkoxysilan mit mindestens einer weiteren funktionellen Gruppe enthält.

Die eingesetzten Alkoholate werden bisweilen auch als Ester des Titans, Zirkoniums und Hafniums bezeichnet.

Der Alkoholteil der erfindungsgemäß eingesetzten Alkoholate kann von aliphatischen oder aromatischen Alkoholen abgeleitet sein. Bevorzugt sind von aliphatischen C₁- bis C₅-Alkoholen abgeleitete Alkoholate, wie insbesondere Tetrabutyltitanat, Tetrapropyltitanat, Tetraisopropyltitanat oder Zirkonium(IV)propylat.

Als weitere funktionelle Gruppe kommen dabei insbesondere eine Vinyl-, Methacryl-, Acryl-, Glycidyl- oder Aminogruppe in Frage, wobei auch Kombinationen von unterschiedlichen funktionellen Gruppen auftreten können. Die Alkoxysilane enthalten in der Regel 1 bis 3 Alkoxygruppen, wobei Methoxy und Ethoxy bevorzugt sind. Spezielle Beispiele besonders geeigneter Alkoxysilane sind Vinyltrimethoxysilan, γ-Methacryloxypropyltrimethoxysilan, 3-Glycidoxypropyltriethoxysilan und Aminoethylaminopropyltrimethoxysilan.

Schließlich hat es sich als geeignet erwiesen, daß die Zusammensetzung auch (c) ein Lösungsmittel für das Alkoholat (a) und für das funktionelle Alkoxysilan (b) enthält. Angesichts der hohen Reaktivität der Alkoholate mit Wasser sind wasserfreie Lösungsmittel bevorzugt. Es kommen dabei insbesondere Aceton, Essigsäureethylester, Isopropanol oder Hexan in Frage.

Das Alkoholat (a) und das funktionelle Alkoxysilan (b) können gemeinsam, z.B. in Form einer Einkomponentenlösung, oder nacheinander, d.h. in Form von zwei Lösungen, aufgebracht werden. Das Alkoholat und das Alkoxysilan können zusammen in Form einer Verpackungseinheit vorliegen, in der beide Komponenten getrennt voneinander vorhanden sind. Aus einer solchen Einheit können die beiden Komponenten unmittelbar vor dem Gebrauch freigesetzt und z.B. zu einer Einkomponent-Lösung gemischt werden. Besonders bevorzugte Lösungen sind wie folgt zusammengesetzt:
- Lösung 1:: 5 bis 50 ml eines Titanalkoholats oder Zirkoniumalkoholats 100 ml Lösungsmittel
- Lösung 2:: 5 bis 10 ml funktionelles Alkoxysilan 100 ml Lösungsmittel.

Durch Mischen der Lösungen 1 und 2 kann eine als Einkomponentenlösung einsetzbare Lösung 3 hergestellt werden.
- Lösung 3:: 5 bis 50 ml Titanalkoholat oder Zirkoniumalkoholat
5 bis 10 ml funktionelles Alkoxysilan 200 ml Lösungsmittel.

Es hat sich gezeigt, daß die erfindungsgemäße Zusammensetzung sich ausgezeichnet zur Verbesserung des Verbundes zwischen Kunststoff und anderen Werkstoffen, wie insbesondere Metallen, einschließlich Legierungen, Keramiken, Glaskeramiken oder Gläsern eignet.

Aus diesem Grund wird sie auch bevorzugt als Dentalmaterial eingesetzt. Mit ihrer Hilfe ist es möglich, sehr unterschiedlich zusammengesetzte Dentallegierungen mit einer Verbundschicht auszustatten, die durch ihre haftvermittelnde Wirkung zu einer sehr guten Anbindung der Dentallegierung an Dentalkunststoffe führt. Neben der Herstellung von z.B. Kunststoff-Metall-Verbunden sind auch Metall-Metall-Verklebungen möglich, wobei beide Metallwerkstoffe mit der Zusammensetzung versehen und durch Einsatz eines Dentalklebstoffes miteinander verbunden werden. Die erhaltenen Verbunde zeichnen sich durch hohe Festigkeit selbst nach häufiger Temperaturwechselbelastung und längerer Wasserlagerung bei erhöhten Temperaturen aus.

Die erfindungsgemäße Zusammensetzung kann ebenfalls bevorzugt als Haftvermittler für Lackbeschichtungen im Maschinen- und Karosseriebau eingesetzt werden.

Bei der Herstellung von Verbunden zwischen Kunststoff und anderen Werkstoffen wird in der Weise vorgegangen, daß
(i) die erfindungsgemäße Zusammensetzung auf den Werkstoff aufgebracht und das Alkoholat hydrolysiert wird und
(ii) der Kunststoff auf den mit der Zusammensetzung versehenen Werkstoff aufgebracht wird.

Das Alkoholat und das Alkoxysilan mit mindestens einer weiteren funktionellen Gruppe können entweder gemeinsam, z.B. in Form einer Einkomponentenlösung, oder nacheinander, z.B. in Form von getrennten Lösungen aufgetragen werden. In letzterem Fall wird bevorzugt zuerst das Alkoholat und erst danach das Alkoxysilan aufgebracht.

Nach Aufbringung der Zusammensetzung in Stufe (i) auf den Werkstoff erfolgt Hydrolyse und damit auch Kondensation der hochreaktiven Titan-, Zirkonium- oder Hafniumalkoholate durch die an der Oberfläche des Werkstoffes vorhandenen OH-Gruppen oder das auf der Oberfläche chemisorptiv gebundene Wasser. Normaler Luftfeuchtigkeit ausgesetzte Werkstoffe haben regelmäßig einen Gehalt an Oberflächen-OH-Gruppen oder chemisorptiv gebundenem Wasser, der ausreicht, damit die Hydrolyse der Alkoholate in ausreichendem Umfang stattfindet. Es bildet sich durch die Hydrolyse und Kondensation schließlich eine an der Werkstoffoberfläche fest haftende TiO₂- ZrO₂- oder HfO₂-haltige Schicht.

Zur Vervollständigung dieser Reaktion und zur Entfernung des durch die Reaktion entstandenen Wassers und Alkohols sowie ggf. vorhandenen Lösungsmittels erfolgt nach Aufbringen der Zusammensetzung eine Wärmebehandlung bei einer Temperatur von insbesondere weniger als 300°C und vorzugsweise im Temperaturbereich von 100 bis 200°C. Üblicherweise wird die Temperaturbehandlung erst dann durchgeführt, wenn etwaiges Lösungsmittel im wesentlichen verdunstet ist.

Durch den gleichzeitigen oder nachfolgenden Einsatz des funktionellen Alkoxysilans (b) erfolgt Silanisierung der gebildeten TiO₂-, ZrO₂- oder HfO₂-haltigen Schicht. Die Funktionalität des Alkoxysilans ermöglicht dabei die Anbindung an die Matrix des in Stufe (ii) aufgebrachten Kunststoffes. Es ist ferner möglich, die Funktionalität des Alkoxysilans an den jeweils ausgewählten Kunststoff anzupassen. Besonders bevorzugte Kunststoffe sind im Dentalbereich eingesetzte Kunststoffe und insbesondere Kunststoffe auf Basis von Acrylaten oder Methacrylaten. Der Kunststoff wird üblicherweise durch Auftragen einer entsprechenden Monomermischung und deren Härtung aufgebracht.

Gegenüber dem Stand der Technik werden mit der erfindungsgemäßen Zusammensetzung und dem erfindungemäßen Verfahren die folgenden Vorteile erzielt:
- die aufgebrachte anorganische Verbundschicht verhindert eine den Verbund schwächende Wasserdiffusion zur Oberfläche des Werkstoffes,
- das Verfestigen der Verbundschicht kann bei Raumtemperatur oder durch Temperaturbehandlung bei Temperaturen von bis zu 200°C erfolgen, so daß keine den Verbund schwächende Veränderung, z.B. an Legierungsoberflächen, auftritt.
- Das erfindungsgemäße Verbundverfahren kann unabhängig von der Zusammensetzung von Legierungen verwendet werden.
- Das erfindungsgemäße Verfahren kann mit nur geringem apparativen Aufwand, wie einfachen Wärmequellen, realisiert werden.

Schließlich bezieht sich die Erfindung auch auf einen Werkstoff auf Basis von Metall, Keramik, Glaskeramik oder Glas, welcher eine Verbundschicht aufweist, die durch die vorstehend beschriebene Aufbringung der erfindungsgemäßen Zusammensetzungen auf die Oberfläche des Werkstoffes und Hydrolyse des Alkoholats (a) erhältlich ist. Derartige Werkstoffe sind für eine spätere sichere Verbindung mit Kunststoff in ausgezeichneter Weise vorbereitet.

Weitere vorteilhafte Ausgestaltungen der Erfindung sowie insbesondere ihr Einsatz in der Dentaltechnik werden in den folgenden Beispielen erläutert.

### Beispiele

Die im folgenden angegebenen Druck-Scher-Festigkeitswerte von Legierungs-Kunstoff-Verbunden oder Legierungs-Legierungs-Verklebungen zeigen die Leistungsfähigkeit der vorliegenden Erfindung. Als Vergleichswert für die Verbundfestigkeit dient die unbehandelte Oberfläche (Leerwert). Bei allen Beispielen wurde entsprechend dem Stand der Technik die Oberfläche der eingesetzten Werkstoffe korundgestrahlt (Korund-Teilchengröße: 50 bis 250 µm, Strahldruck: 1 bis 5 bar).

Die eingesetzten haftvermittelnden Lösungen 1, 2 und 3 setzten sich wie folgt zusammen:
- Lösung 1:: 100 ml Tetraisopropyltitanat
900 ml Essigsäureethylester
- Lösung 2:: 100 ml γ-Methacryloxypropyltrimethoxysilan
900 ml Aceton
- Lösung 3:: 100 ml Tetraisopropyltitanat
400 ml γ-Methacryloxypropyltrimethoxysilan
500 ml Essigsäureethylester

### Beispiel 1 - Legierungs-Kunststoff-Verbund

Zunächst wurde Lösung 1 auf die korundgestrahlte Oberfläche einer ausgewählten Legierung aufgetragen. Nach dem Verdunsten des Lösungsmittels erfolgte der Auftrag von Lösung 2. Die Legierung mit den beiden Schichten wurde anschließend entweder einem längeren Trocknungsprozeß bei 25 bis 80°C, vorzugsweise 30 Minuten bei 50°C, oder einer kürzeren Temperaturbehandlung bei 100 bis 200°C, vorzugsweise 2 Minuten bei 150°C, unterworfen. Bei Verwendung der Einkomponentenlösung 3 anstelle von Lösung 1 und 2 wurde nach dem Aufbringen dieser Lösung in gleicher Weise getrocknet und getempert.

Dann wurde auf die erhaltene Verbundschicht ein handelsüblicher Dentalopaker auf Methacrylat-Basis aufgebracht und polymerisiert und danach der eigentliche Verblendkunststoff mittels eines Metallringes (Durchmesser: 5 mm, Höhe: 2 mm) aufmodelliert und ebenfalls polymerisiert. Nach dem Abziehen des Metallringes befand sich ein Kunststoffzylinder mit einem Durchmesser von 5 mm und einer Höhe von 2 mm auf der Legierung. Der so hergestellte Metall-Kunststoff-Verbund wurde 24 Stunden in destilliertem Wasser gelagert und danach in einem Druck-Scher-Versuch auf Verbundfestigkeit nach ISO-Norm 10477 getestet (Vorschubgeschwindigkeit der Prüfmaschine: 1 mm min⁻¹. Um die über die Jahre erfolgenden tatsächlichen mechanischen Belastungen im Mund simulieren zu können, wurden die hergestellten Verbunde zusätzlich einer Temperaturwechselbelastung (TWL) unterzogen oder drei Tage lang gekocht. Bei der Temperaturwechselbelastung erfolgten 5000 Temperaturwechsel zwischen den Temperaturen t₁ = 5°C und t₂ = 55°C. Die gemessenen Verbundfestigkeiten für verschiedene Dentallegierungen und Dentalkunststoffe sind in den Tabellen I und II angegeben.

Dabei stehen die angegebenen Bezeichnungen für folgende Materialien:

| | | |
|---|---|---|
| Albabond | = | Palladiumlegierung |
| Mainbond EH | = | Goldlegierung |
| Maingold SG | = | Goldlegierung |
| Hera GG | = | Goldlegierung |
| Titan | = | Titanlegierung |
| Heraenium CE | = | Kobalt-Chrom-Legierung |
| Wiron 88 | = | Nickel-Legierung |

Bei den Dentalkunststoffen handelte es sich um handelsübliche Materialien auf Basis von Dimethacrylaten.

**Tabelle I Legierungs-Kunststoff-Verbund Opaker: Art-Glas Opaker (Fa. Kulzer), Verblendkunststoff: Art-Glas (Fa. Kulzer))**

| Dentallegierung | | | | Scherfestigkeit [MPa] | | | | |
|---|---|---|---|---|---|---|---|---|
| | Leerwert | | | | Lösung 1 + Lösung 2; 150°C, 2 min | | | |
| | -24 h, 37°C | 5000, TWL | 1d gekocht | 3d gekocht | 24 h, 37°C | 5000, TWL | 1d gekocht | 3d gekocht |
| Albabond | 8,6 | 5,8 | 2,1 | 1,4 | 25,7 | 23,9 | 22,1 | 14,3 |
| Mainbond EH | 10,4 | 6,2 | 2,4 | 1,1 | 28,3 | 27,6 | 23,8 | 16,7 |
| Maingold SG | 10,0 | 6,4 | 2,0 | 0,8 | 27,1 | 25,8 | 21,3 | 15,1 |
| Hera GG | 9,8 | 6,0 | 2,2 | 1,3 | 26,8 | 25,3 | 20,7 | 14,5 |
| Titan | 11,0 | 6,5 | 1,8 | 1,1 | 28,7 | 27,0 | 23,4 | 16,9 |
| Heraenium CE | 9,2 | 5,8 | 1,5 | 0,7 | 26,0 | 24,3 | 21,0 | 13,7 |
| Wiron 88 | 8,8 | 5,5 | 1,6 | 0,8 | 25,3 | 23,8 | 20,2 | 13.2 |

**Tabelle II Legierungs-Kunststoff-Verbund (Metallegierung: Mainbond EH; Opaker: Visto-Gem-Opaker (Fa. Espe))**

| Dentalkunststoff | | | | Scherfestigkeit [MPa] | | | | |
|---|---|---|---|---|---|---|---|---|
| | Leerwert | | | | Lösung 1 + Lösung 2; 150°C, 2 min | | | |
| | 24 h, 37°C | 5000, TWL | 1d gekocht | 3d gekocht | 24 h, 37°C | 5000, TWL | 1d gekocht | 3d gekocht |
| Visio-Gem (Fa. Espe) | 8,2 | 6,5 | 2,1 | 1,1 | 24,2 | 21,5 | 19,2 | 13,4 |
| Art-Glass (Fa. Kulzer) | 10,8 | 7,8 | 2,6 | 1,7 | 29,1 | 28,3 | 24,0 | 16,2 |
| Tetric (Fa. Vivadent) | 11,2 | 9,2 | 2,2 | 1,5 | 27,6 | 26,0 | 21,6 | 14,5 |
| Z 100 (Fa. 3M) | 12,0 | 9,5 | 2,9 | 1,9 | 29,8 | 28,0 | 25,2 | 17,0 |
| Prodigy (Fa. Voco) | 9,2 | 7,3 | 2,0 | 1,8 | 26,7 | 25,0 | 22,1 | 14,2 |
| Polofil | 8,2 | 6,8 | 1,7 | 1,3 | 25,5 | 22,1 | 21,0 | 13,5 |

Die Druck-Scherfestigkeitsmessungen der Legierungs-Kunststoff-Verbunde (Tabellen I und II) zeigen, daß nach der 24-stündigen Wasserlagerung bei 37°C und den 5000 Wechsellastzyklen mit den erfindungsgemäßen Lösungen eine fast 200 %ige Steigerung der Verbundfestigkeit im Vergleich zu unbehandelten, nur korundgestrahlten Legierungsoberflächen erzielt wird. Der Unterschied zwischen behandelter Oberfläche zu unbehandelter Oberfläche wird noch deutlicher, wenn die Legierung-Kunststoff-Verbunde dem extremen Kochtest unterzogen werden. An der lediglich korundgestrahlten Oberfläche lag mit 1 bis 2 MPa eine nur noch äußerst geringe Haftung des Kunststoffes an der Legierungsoberfläche vor, während mit der erfindungsgemäßen Lösung noch ausreichend hohe Haftwerte von 13 bis 17 MPa gemessen wurden.

Die Auflistung der sehr unterschiedlich zusammengesetzten Dentallegierungen in Tabelle I von hochgoldhaltigen Legierungen bis zu Legierungen von unedlen Metallen macht zudem deutlich, daß die haftvermittelnde Wirkung der hergestellten Verbundschicht von der Zusammensetzung der Legierung unabhängig ist.

### Beispiel 2 - Legierungs-Legierungs-Verklebung

In diesem Beispiel wurden Dentallegierungen unter Zuhilfenahme von Dentalkunststoffen miteinander verklebt. Die Vorbereitung der Legierungsoberflächen erfolgte analog zu der in Beispiel 1 beschriebenen Weise. Die Überlappungslänge der Verklebung betrug 10 mm und die Klebefläche war 100 mm². Die verklebten Legierungsproben wurden bis zu 5 Tage lang gekocht. Die ermittelten Scherfestigkeiten sind in der nachstehenden Tabelle III angegeben.

Bei Chemiace, Brilliant enamel kit, Palavit 55 und Twinlook handelt es sich um kommerzielle Dentalkunststoffe. Pattex Stabilit ist ein handelsüblicher Allzweckkleber.

**Tabelle III Legierungs-Legierungs-Verklebung (Metallegierung: Wiron 88)**

| **Dentalkunststoff** | | **Scherfestigkeit [MPa]** | | |
|---|---|---|---|---|
| | **Leerwert** | | **Lösung 1 + Lösung 2, 150°C, 4 min** | |
| | 24 h, 37°C | 5d gekocht | 24 h, 37°C | 5d gekocht |
| Chemiace (Fa. Sun Medical) | 12,3 | 5,5 | 22,4 | 18,9 |
| Brilliant enamel kit (Fa. Colttne) | 11,8 | 4,4 | 20,1 | 16,8 |
| Palavit 55 (Fa. Kulzer) | 10,2 | 4,8 | 21,2 | 18,0 |
| Twinlook (Fa. Kulzer) | 11,4 | 5,1 | 22,6 | 18,9 |
| | | | | |
| Pattex Stabilit (Epoxidharz) (Fa. Henkel) | 13,4 | 6,8 | 24,7 | 20,7 |

Die in Tabelle III aufgeführten Meßwerte zeigen ein analoges Bild wie für die Legierungs-Kunststoff-Verbunde. Durch die Metall-Metall-Verklebung kann der Wasserzutritt nur über die dünne Klebefuge (50-150 µm) erfolgen. An der unbehandelten Oberfläche fallen die Verbundwerte durch das Kochen auf die Hälfte ab. Mit der erfindungsgemäßen Lösung wurde bei den Verklebungen gegenüber der unbehandelten Oberfläche die doppelte Verbundfestigkeit erreicht. Durch die Kochbehandlung erfolgte ein nur geringfügiger Abfall der Haftfestigkeit um etwa 20 %.

Durch die Erfindung steht eine haftfeste, feuchtigkeitsstabile und hydrolysebeständige Verbundschicht zur Verfügung, die sich für die vielfältigsten Verbunde eignet und nicht nur auf die Anwendung in der Dentaltechnik beschränkt ist. Eine ebenso vorteilhafte Verwendung der erfindungsgemäßen Lösung ist zur Herstellung von Verbundschichten für Lackbeschichtung im Maschinen- und Karosseriebau. Auch unter extremen feuchten Bedingungen wird damit die Haltbarkeit einer Lackbeschichtung oder -verklebung deutlich verlängert.

## Patentansprüche

1. Haftvermittelnde Zusammensetzung für Verbunde zwischen Kunststoff und anderen Werkstoffen, **dadurch gekennzeichnet, daß** sie
(a) mindestens ein Alkoholat des Titans, des Zirkoniums oder des Hafniums und
(b) mindestens ein Alkoxysilan mit mindestens einer weiteren funktionellen Gruppe
enthält.

2. Zusammensetzung nach Anspruch 1, **dadurch gekennzeichnet, daß** sie als Alkoholat (a) Tetrabutyltitanat, Tetrapropyltitanat, Tetraisopropyltitanat und/oder Zirconium(IV)propylat enthält.

3. Zusammensetzung nach Anspruch 1 oder 2, **dadurch gekennzeichnet, daß** das Alkoxysilan (b) als weitere funktionelle Gruppe eine Vinyl-, Methacryl-, Acryl-, Glycidyl- oder Aminogruppe aufweist.

4. Zusammensetzung nach Anspruch 3, **dadurch gekennzeichnet, daß** sie als Alkoxysilan (b) Vinyltrimethoxysilan, γ-Methacryloxypropyltrimethoxysilan, 3-Glycidoxypropyltriethoxysilan oder Aminoethylaminopropyltrimethoxysilan enthält.

5. Zusammensetzung nach einem der Ansprüche 1 bis 4, **dadurch gekennzeichnet, daß** sie in Form einer Verpackungseinheit vorliegt, in der getrennt voneinander das Alkoholat (a) und das Alkoxysilan (b) vorhanden sind.

6. Zusammensetzung nach einem der Ansprüche 1 bis 5, **dadurch gekennzeichnet, daß** sie
(c) ein Lösungsmittel
enthält.

7. Zusammensetzung nach Anspruch 6, **dadurch gekennzeichnet, daß** sie ein wasserfreies Lösungsmittel für das Alkoholat (a) enthält.

8. Zusammensetzung nach Anspruch 6 oder 7, **dadurch gekennzeichnet, daß** das Lösungsmittel (c) Aceton, Essigsäureethylester, Isopropanol oder Hexan ist.

9. Verwendung der Zusammensetzung gemäß einem der Ansprüche 1 bis 8 zur Verbesserung des Verbunds zwischen einerseits Kunststoff und andererseits Metall, Keramik, Glaskeramik oder Glas.

10. Verwendung nach Anspruch 9, wobei die Zusammensetzung als Dentalmaterial oder als Haftvermittler für Lackbeschichtungen im Maschinen- und Karosseriebau eingesetzt wird.

11. Verfahren zu Herstellung von Verbunden zwischen Kunststoff und anderen Werkstoffen, bei dem
(i) eine Zusammensetzung gemäß einem der Ansprüche 1 bis 8 auf den Werkstoff aufgebracht und das Alkoholat hydrolysiert wird und
(ii) der Kunststoff auf den mit der Zusammensetzung versehenen Werkstoff aufgebracht wird.

12. Verfahren nach Anspruch 11, bei dem ein Kunststoff auf Basis von Acrylaten oder Methacrylaten eingesetzt wird.

13. Verfahren nach Anspruch 11 oder 12, bei dem ein Metall, eine Keramik, eine Glaskeramik oder ein Glas und insbesondere eine Dentallegierung als anderer Werkstoff eingesetzt wird.

14. Verfahren nach einem der Ansprüche 11 bis 14, bei dem nach Stufe (i) die Zusammensetzung einer Wärmebehandlung unterworfen wird.

15. Verfahren nach Anspruch 14, bei dem die Wärmebehandlung bei einer Temperatur von 100 bis 200°C durchgeführt wird.

16. Verfahren nach einem der Ansprüche 11 bis 15, bei dem in Stufe (i) zuerst das Alkoholat und danach das Alkoxysilan aufgebracht wird.

17. Werkstoff auf Basis von Metall, Keramik, Glaskeramik oder Glas, **dadurch gekennzeichnet, daß** er eine Verbundschicht aufweist, die durch Aufbringen der Zusammensetzung gemäß einem der Ansprüche 1 bis 8 auf die Oberfläche des Werkstoffes und Hydrolyse des Alkoholats (a) erhältlich ist.

18. Verfahren zum Aufbringen einer Verbundschicht auf einen Werkstoff auf Basis von Metall, Keramik, Glaskeramik oder Glas, **dadurch gekennzeichnet, daß**
(1) mindestens ein Alkoholat des Titans, des Zirkoniums oder des Hafniums auf die Oberfläche des Werkstoffs aufgebracht und hydrolysiert wird und anschließend
(2) mindestens ein Alkoxysilan mit mindestens eines weiteren funktionellen Gruppe auf die Oberfläche des Werkstoffs aufgebracht wird.

## Claims

1. Adhesion-promoting composition for bonds between plastic and other materials, **characterised in that** said composition comprises
(a) at least one alcoholate of titanium, zirconium or hafnium and
(b) at least one alkoxy silane having at least one additional functional group.

2. Composition according to Claim 1, **characterised in that** it comprises tetrabutyl titanate, tetrapropyl titanate, tetraisopropyl titanate or zirconium (IV) propylate as the alcoholate (a).

3. Composition according to Claim 1 or 2, **characterised in that** the alkoxy silane (b) possesses a vinyl, methacryl, acryl, glycidyl or amino group as the additional functional group.

4. Composition according to Claim 3, **characterised in that** it comprises vinyl trimethoxy silane, ?-methacryloxypropyl trimethoxy silane, 3-glycidoxypropyl triethoxy silane or aminoethylaminopropyl trimethoxy silane as the alkoxy silane (b).

5. Composition according to one of Claims 1 to 4, **characterised in that** it is present in the form of a packaging unit in which the alcoholate (a) and the alkoxy silane (b) are separated from each other.

6. Composition according to one of Claims 1 to 5, **characterised in that** it comprises
(c) a solvent.

7. Composition according to Claim 6 **characterised in that** it comprises an anhydrous solvent for the alcoholate (a).

8. Composition according to Claim 6 or 7, **characterised in that** the solvent (c) is acetone, ethyl acetate, isopropanol or hexane.

9. Use of the composition according to one of Claims 1 to 8 for improving the bond between plastic on the one hand and metal, ceramic, glass ceramic or glass on the other hand.

10. Use according to Claim 9, wherein the composition is used as a dental material or as an adhesion promoter for paint coatings in machine construction and vehicle body construction.

11. Process for the production of bonds between plastic and other materials, whereby
(i) a composition according to one of Claims 1 to 8 is applied to the material and the alcoholate is hydrolysed and
(ii) the plastic is applied to the material that has been provided with the composition.

12. Process according to Claim 11, whereby a plastic based on acrylates or methacrylates is used.

13. Process according to Claim 11 or 12, whereby a metal, a ceramic, a glass ceramic or a glass and in particular a dental alloy is used as the other material.

14. Process according to one of Claims 11 to 13, whereby the composition is subjected to a heat treatment after step (i).

15. Process according to Claim 14, whereby the heat treatment is carried out at a temperature from 100 to 200 °C.

16. Process according to one of Claims 11 to 15, whereby first the alcoholate and then the alkoxy silane is applied in step (i).

17. Material based on metal, ceramic, glass ceramic or glass, **characterised in that** it has a bonding layer that is obtainable by applying the composition according to one of Claims 1 to 8 onto the surface of the material and hydrolysis of the alcoholate (a).

18. Process for applying a bonding layer onto a material based on metal, ceramic, glass ceramic or glass, **characterised in that**
(1) at least one alcoholate of titanium, zirconium or hafnium is applied onto the surface of the material and hydrolysed, and subsequently
(2) at least one alkoxy silane having at least one additional functional group is applied to the surface of the material.

## Revendications

1. Composition compatibilisante pour liaison entre un plastique et d'autres matériaux, **caractérisée en ce qu'**elle contient :
(a) au moins un alcoolate de titane, de zirconium ou de hafnium,
(b) au moins un alcoxysilane avec au moins un autre groupe fonctionnel.

2. Composition selon la revendication 1, **caractérisée en ce qu'**elle contient comme alcoolate (a) du tetrabutyltitanate, du tetrapropyltitanate, du tetraisopropyltitanate et/ou du propylate de zirconium (IV).

3. Composition selon la revendication 1 ou 2, **caractérisée en ce que** l'alcoxysilane (b) présente comme autre groupe fonctionnel un groupement vinyle, méthacryle, acryle, glycidyle ou amine.

4. Composition selon la revendication 3, **caractérisée en ce que**lle contient comme alcoxysilane (b) du vinyltriméthoxysilane, du γ-méthacryloxypropyltriméthoxysilane, du 3-glycidoxypropyltriéthoxysilane ou du aminoéthylaminopropyltriméthoxysilane.

5. Composition selon une des revendications 1 à 4, **caractérisée en ce qu'**elle est présente sous forme d'un emballage unique dans lequel l'alcoolate (a) et l'alcoxysilane (b) sont présents séparés l'un de l'autre.

6. Composition selon une des revendications 1 à 5, **caractérisée en ce qu'**elle contient :
(c) un solvant.

7. Composition selon la revendication 6, **caractérisée en ce qu'**elle contient un solvant anhydre pour l'alcoolate (a).

8. Composition selon une des revendications 6 ou 7, **caractérisée en ce que** le solvant (c) est de l'acétone, de l'acétate d'éthyle, de l'isopropanol ou de l'hexane.

9. Utilisation de la composition selon une des revendications 1 à 8 en vue de l'amélioration de la liaison entre d'une part un plastique et d'autre part un métal, une céramique, une vitrocéramique ou un verre.

10. Utilisation selon la revendication 9, où la composition est utilisée comme matériau dentaire ou comme agent compatibilisant pour des revêtements de vernis dans la construction de machines ou de carrosseries.

11. Procédé de fabrication de liaisons entre un plastique et d'autres matériaux, où :
(i) une composition selon une des revendications 1 à 8 est appliquée sur le matériau et l'alcoolate est hydrolysé, et
(ii) le plastique est appliqué sur le matériau pourvu de la composition.

12. Procédé selon la revendication 11, où est mis en oeuvre un plastique à base d'acrylates ou de methacrylates.

13. Procédé selon la revendication 11 ou 12, où est utilisé un métal, une céramique, une vitrocéramique ou un verre et en particulier un alliage dentaire comme autre matériau.

14. Procédé selon une des revendications 11 à 14, où après l'étape (i) la composition est soumise à un traitement thermique.

15. Procédé selon la revendication 14, où le traitement thermique est effectué à une température de 100 à 200°C.

16. Procédé selon une des revendications 11 à 15, où à l'étape (i) est appliqué d'abord l'alcoolate puis l'alcoxysilane.

17. Matériau à base de métal, de céramique, de vitrocéramique ou de verre, **caractérisé en ce qu'**il présente une couche de liaison susceptible d'être obtenue par application de la composition selon une des revendications 1 à 8 sur la surface du matériau et par hydrolyse de l'alcoolate (a).

18. Procédé d'application d'une couche de liaison sur un matériau à base de métal, de céramique, de vitrocéramique ou de verre, **caractérisé en ce que** :
(1) au moins un alcoolate de titane, de zirconium ou de hafnium est appliqué sur la surface du matériau et hydrolysé, puis
(2) au moins un alcoxysilane avec au moins un autre groupe fonctionnel est appliqué sur la surface du matériau.
